# EUROPÄISCHE PATENTSCHRIFT

(11) **EP 2 066 288 B1**
(45) Veröffentlichungstag und Bekanntmachung des Hinweises auf die Patenterteilung: **21.11.2012**
(21) Anmeldenummer: 07802333.0
(22) Anmeldetag: 15.09.2007
(51) Int. Cl.: A61K 8/11, A61K 8/20, A61K 8/23, A61K 8/25, A61K 8/37, A61K 8/60, A61K 8/73, A61K 8/81, A61K 8/85, A61K 8/92, A61K 8/97, A61Q 19/10

(54) **PEELINGKAPSELN**
PEELING CAPSULES
CAPSULES POUR GOMMAGE

(30) Priorität: 22.09.2006 DE 102006044942
(43) Veröffentlichungstag der Anmeldung: 10.06.2009
(73) Patentinhaber: Beiersdorf AG, 20253 Hamburg (DE)
(72) Erfinder: SCHÄFER, Jessica, 22299 Hamburg (DE); WEYLAND, Silke, 87551 Worms (DE)
(86) Internationale Anmeldenummer: PCT/EP2007/008051
(87) Internationale Veröffentlichungsnummer: WO 2008/034565

(56) Entgegenhaltungen:
- EP-A- 0 330 453
- WO-A-00/04867
- WO-A-2005/020940
- JP-A- 63 185 914
- US-A1- 2006 127 427

## Beschreibung

Die Erfindung betrifft kosmetische und dermatologische Peelingzubereitungen in Form von Kapseln umfassend eine formstabile Kapselhülle und eine Füllung.

Die Epidermis setzt sich aus unterschiedlichen Zelltypen zusammen: Die Keimschicht (Stratum germinativum), die Körnerschicht (Stratum granulosum) und die Hornschicht (Stratum corneum). In der Keimschicht werden durch Teilung neue Zellen gebildet, die permanent an die Hautoberfläche wandern. Auf dem Weg dorthin, in der Körnerschicht, verhornen sie und sterben ab. Diese abgestorbenen Hornzellen machen als Hornsäulen den Großteil der Zellen in der Haut aus. Innerhalb von 28 Tagen hat sich die Epidermis einmal erneuert. Meistens lösen sich die toten Hornzellen nach und nach unmerklich und von selbst ab.

An stark beanspruchten Körperstellen, wie z.B. Hände, Füße oder Knie aber auch bei sehr trockener Haut können sich vermehrt Hornschuppen ansammeln, die sich nicht von selbst lösen und so zu unschöner, matter und teils sogar ausgefranster Haut führen.

Es gibt verschiedene Möglichkeiten um die Haut von diesen überschüssigen Hornschuppen zu befreien. Zum einen gibt es verschiedene Werkzeuge, wie Bimssteine, Waschlappen oder Luffawurzeln. Zum anderen ist es weit verbreitet die Haut mit losen Körnern, wie z.B. Sand, Salz, Zucker oder Weizenschrot abzureiben. Ferner gibt es kosmetische Zubereitungen, wie Waschpeelings, die meist abrasiv wirkende Kunststoffpartikel enthalten. Um diese Kunststoffpartikel mühelos wieder von der Haut entfernen zu können, sind den meisten Waschpeelings Tenside zugesetzt. Diese Tenside entfernen jedoch nicht nur die Kunststoffpartikel, sondern auch Hauteigene Lipide, die sich auf der Haut befinden und führen nach der Anwendung somit häufig zu einem spannen der Haut.

Dem spannenden Hautgefühl kann entgangen werden, indem der kosmetischen Peelingzubereitung gleich Öle oder Lipide zugesetzt werden. Ein Beispiel für besonders rückfettende Peelingzubereitungen sind Öl/Salzgemische.

Salzaufschlämmungen in Öl zur Verwendung als Peelingzubereitung sind dem Verbraucher seit einiger Zeit bekannt (Beispiel: "Großmutters Hausmittel, neu entdeckt", © 2000 Reader's Digest, Verlag Das Beste GmbH, Stuttgart, Zürich, Wien). Solche Mischungen sind ebenfalls als kosmetische Fertigprodukte im Handel erhältlich (Beispiel: Alessandro® Hands!Up Magic Manicure®). Die feinen Salzpartikel in diesen Gemischen setzen sich mit der Zeit am Boden des Behälters ab und bilden dort eine sichtbare Salzschicht. Das Vermischen der beiden Phasen kann je nach Partikelgröße des eingesetzten Salzes sehr zeit- und kraftaufwendig sein. Oftmals ist es nicht möglich eine gleichmäßige Durchmischung des Produktes zu erreichen was zur Folge hat, dass das Öl in einem schnelleren Umfang aufgebraucht wird. Zurück bleibt dann eine nahezu trockene und feste Salzschicht auf dem Boden des Behälters, welche nicht mehr verwendet werden kann.

Ansätze um diesen Missstand zu beheben werden in den Schriften WO 00/04867 und US 5,866,145 Emulsionskapseln vorgestellt.

Im Rahmen dieser Erfindung soll das Problem der Sedimentation gelöst werden, indem die Salzaufschlämmung als Einmalanwendung in eine Kapsel eingebunden wird..

Kosmetische Kapseln als Darreichungsform kosmetischer Wirkstoffe - oder auch lediglich als Zubereitungsform mit eigentümlichem Anwendungserlebnis - sind als solche ebenfalls bekannt. Sie werden beispielsweise in den Schriften WO 05/20940 und WO 05/20949 beschrieben und als Emulsionskapseln bezeichnet.

Der Nachteil solcher Emulsionskapseln ist, daß die Kapselhülle oftmals in unschöner Weise in groben Brocken auf der Haut verschmiert.

Es war indes nicht vom Stande der Technik nahegelegt, daß kosmetische Peelingzubereitungen in Kapselform aus
- einer Kapselhülle aus
- der Gruppe der bei 25 °C festen kosmetisch üblichen Wachse, oder der bei 25 °C festen Mischungen aus Wachsen und einer oder mehreren Substanzen gewählt aus der Gruppe der kosmetisch üblichen Lipide, Wachse , Emulgatoren und natürlichen oder synthetischen Polymere,
- wobei die Wachse gewählt werden aus aus der Gruppe Cetylpalmitat, Cetylrizinoleat, Bienenwachs, hydrierte Kokosglyceride, Methylpalmitat, Candelillawachs, Camaubawachs, Paraffinwachs, Ceresin, Ozokerit, Myristylmyristat, Tripalmitin. Tribehenin, Glycerylpalmitostearat, hydriertes Rapsöl und/oder C15-C40 Alkylstearylstearat, bevorzugt Ceresin und/oder Ozokerit,
- und einer Kapselfüllung aus
- einem Öl oder Lipidmischung welches oder welche bei 25 °C eine Viskosität zwischen 3 und 50 000 m Pas, bevorzugt zwischen 1000 und 8000 und ganz besonders bevorzugt zwischen 2000 und 4000 mPas aufweist, und einer oder mehreren abrasiv wirkenden Festkörpersubstanzen, gewählt aus der Gruppe Kochsalz, Steinsalz, Siedesalz, Meersalz oder Himalayasalz.
die Probleme des Standes der Technik lösen würde.

Bei der Anwendung wird die gesamte Kapsel verrieben, sowohl Hülle als auch Füllung. Durch die abrasiv wirkenden Festkörpersubstanz oder -Substanzen in der Füllung erhält man einen Peelingeffekt, die Öle sorgen zum einen für ein gutes Gleiten der abrasiv wirkenden Festkörpersubstanz oder -Substanzen und zum anderen wirken sie rückfettend.

Die WO 2005/020940 beschreibt zwar kosmetische und/oder dermatologischer Kapseln umfassend eine Kapselhülle, die fest, halbfest und/oder formstabil ist und im Wesentlichen aus Wachsen, Emulgatoren, natürlichen und/oder synthetischen Polymeren und/oder Mischungen daraus besteht.

Dieses Dokument konnte aber nicht den Weg zur vorliegenden Erfindung weisen.

Die abrasiv wirkende Festkörpersubstanz oder -Substanzen im Inneren helfen zudem bei der Auflösung der Kapselhülle. Das bei den Emulsionskapseln zu beobachtende Problem der schlecht zu verteilenden Kapselhülle könne durch den Zusatz der abrasiv wirkenden Festkörpersubstanz oder -Substanzen deutlich verbessert werden.

Die Wachse der Kapselhülle bilden einen Schutzfilm aus. Nach dem gründlichen Einreiben und Verteilen können verbleibende Überreste unter Wasser abgewaschen werden.

Die Eigenschaft der Hülle ist vorteilhaft dadurch gekennzeichnet, daß sie
- beim Verreiben oder Verteilen der Zubereitung auf der Haut schmilzt und/oder aufgrund von Scherkräften völlig oder teilweise flüssig wird
- und/oder sie sich in der Füllung und/oder den Hautsebumlipiden bzw. durch Vermischung von innerer Phase und Hüllmaterial auflöst
und so für den Anwender nicht mehr als gesondert neben der Füllung vorliegender Bestandteil der Zubereitung wahrnehmbar ist.

Viele Begriffe wie "Kugeln", "Kapseln" oder "kapselförmige Zubereitung" können grundsätzlich zur Beschreibung der erfindungsgemäßen Kapseln herangezogen werden, jedoch werden diesen Begriffen unter Umständen verschiedene Bedeutungen zugeordnet.

Generell ist erfindungsgemäß eine Kapsel ein beispielsweise ungefähr runder oder ellipsoider, von seiner Umgebung klar unterscheidbarer Gegenstand, der bei leichtem Druck, also beispielsweise durch Anfassen bei der Entnahme aus einer Verpackung, seine Form nicht oder nicht wesentlich ändert. Im Rahmen dieser Offenbarungsschrift wird dieser Zustand der Kapsel auch als "formstabil" oder "fest" bezeichnet.

Erfindungsgemäß sind aber auch anderen Formen der Kapseln bzw. der Zubereitungen denkbar, sofern die beanspruchten Merkmale der Kapsel, der Kapselhülle und der Füllung eingehalten werden.

Die erfindungsgemäßen Kapseln weisen einen durchschnittlichen Durchmesser von typischerweise 5 bis 50 mm auf und sind bevorzugt etwa erbsen- bis kirschgroß, also ca. 8 bis 15 mm im Durchmesser. Damit sind die Kapseln einzeln handhabbar und anwendbar.

Im Rahmen der vorliegenden Offenbarung wird als Oberbegriff für Fette, Öle, Wachse und dergleichen gelegentlich der Ausdruck "Lipide" verwendet, wie dem Fachmann durchaus geläufig ist. Auch werden die Begriffe "Ölphase" und "Lipidphase" synonym angewandt.

Die innere Ölphase der erfindungsgemäßen Formulierungen wird vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Sojaöl, Erdnussöl,

Rapsöl, Mandelöl, Palmöl, Kokosöl, Rizinusöl, Weizenkeimöl, Traubenkemöl, Distelöl, Nachtkerzenöl, Macadamianußöl und dergleichen mehr.

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether (*Cetiol* OE) und/oder Dicaprylylcarbonat, beispielsweise das unter der Handelsbezeichnung *Cetiol* CC erhältlich ist.

Es ist ferner bevorzugt, die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid zu wählen. Es ist insbesondere vorteilhaft, wenn die Ölphase der erfindungsgemäßen Formulierungen einen Gehalt an C₁₂₋₁₅-Alkylbenzoat aufweist oder vollständig aus diesem besteht.

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene und Isohexadecan. Unter den Polyolefinen sind Polydecene die bevorzugten Substanzen.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Silikonöle sind hochmolekulare synthetische polymere Verbindungen, in denen SiliciumAtome über Sauerstoff-Atome ketten- und/oder netzartig verknüpft und die restlichen Valenzen des Siliciums durch Kohlenwasserstoff-Reste (meist Methyl-, seltener Ethyl-, Propyl-, Phenyl-Gruppen u. a.) abgesättigt sind. Systematisch werden die Silikonöle als Polyorganosiloxane bezeichnet. Die methylsubstituierten Polyorganosiloxane, welche die mengenmäßig bedeutendsten Verbindungen dieser Gruppe darstellen und sich durch die folgende Strukturformel auszeichnen 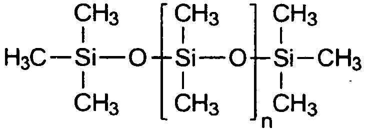 werden auch als Polydimethylsiloxan bzw. Dimethicon (INCl) bezeichnet. Dimethicone gibt es in verschiedenen Kettenlängen bzw. mit verschiedenen Molekulargewichten.

Besonders vorteilhafte Polyorganosiloxane im Sinne der vorliegenden Erfindung sind beispielsweise Dimethylpolysiloxane [Poly(dimethylsiloxan)], welche beispielsweise unter den Handelsbezeichnungen Abil 10 bis 10 000 bei Th. Goldschmidt erhältlich sind. Ferner vorteilhaft sind Phenylmethylpolysiloxane (INCl: Phenyl Dimethicone, Phenyl Trimethicone), cyclische Silikone (Octamethylcyclotetrasiloxan bzw. Decamethylcyclopentasiloxan), welche nach INCl auch als Cyclomethicone bezeichnet werden, aminomodifizierte Silikone (INCl: Amodimethicone) und Silikonwachse, z. B. Polysiloxan-Polyalkylen-Copolymere (INCl: Stearyl Dimethicone und Cetyl Dimethicone) und Dialkoxydimethylpolysiloxane (Stearoxy Dimethicone und Behenoxy Stearyl Dimethicone), welche als verschiedene Abil-Wax-Typen bei Th. Goldschmidt erhältlich sind. Aber auch andere Silikonöle sind vorteilhaft im Sinne der vorliegenden Erfindung zu verwenden, beispielsweise Cetyldimethicon, Hexamethylcyclotrisiloxan, Polydimethylsiloxan, Poly(methylphenylsiloxan).

Die Öle, die für die innere Ölphase verwendet werden können, sind auch vorteilhafte Zusatzstoffe für das Hüllmaterial.

Die bevorzugte abrasiv wirkende Festkörpersubstanz ist kristallines Kochsalz. Aber auch Meersalz, Steinsalz, Siede- und Salinensalz sowie Himalayasalz.

Es ist aber auch vorteilhaft, zusätzlich weitere abrasiv wirkende Festkörpersubstanzen, gewählt aus der Gruppe der festen, abrasiven Partikel aus allen organischen und anorganischen Feststoffen auf natürlicher und synthetischer Basis zu verwenden.

Erfindungsgemäß bevorzugte Füllstoffe kommen aus der Gruppe der anorganischen oder organischen Siliziumverbindungen.

Von den anorganischen Siliziumverbindungen sind besonders bevorzugt die Schichtsilikate. Davon besonders bevorzugt sind Kaolin, Talkum und Mica.

Weiterhin gehören zu den bevorzugten anorganischen Siliziumverbindungen die and der Oberfläche organisch modifizierten sphärischen Partikel.

Von diesen sind besonders bevorzugt die Polymethylsilsesquioxane und hydrophob modifizierte Aerosile, wie z.B. Aerosil R 972.

Zu den organischen Siliziumverbindungen gehören die Siloxan Elastomere und Siloxan Harze. Von denen sind besonders bevorzugt die KSP-Typen von Shin Etsu, sowie das Trimethylsiloxysilicate.

Weitere erfindungsgemäß bevorzugte Füllstoffe kommen aus der Gruppe der sphärischen Partikel. Besonders bevorzugt ist der mittlere Teilchendurchmesser kleiner als 20 µm. Weiterhin werden bevorzugt sphärische Partikel organischen Ursprungs gewählt. Desweiteren sind bevorzugt sphärische Partikel mit einem mittierern Teilchendurchmesser kleiner als 10 µm. Davon besonders bevorzugt sind Nylon-12, das z.B. als SP-501 oder SP-500 von der Firma Kobo vertrieben wird. Weiterhin bevorzugt sind Polymethylmethacrylate, die z.B. unter dem Handelsnamen Covabead LH 85 von LCW vertrieben wird.

Weiterhin bevorzugt werden Stärkederivate eingesetzt. Davon besonders bevorzugt Aluminium Starch Octenylsuccinate, Maisstärke oder Natrium Cornstarch Octenylsuccinate.

Es ist erfindungsgemäß von Vorteil, wenn die Zubereitungen, dadurch gekennzeichnet sind, daß sie 0,001 Gew.-% bis 10 Gew.-%, bevorzugt 0,05 Gew.-% bis 5 Gew.-%, insbesondere 1 - 4 Gew.-%, bezogen auf das Gesamtgewicht der Zubereitungen, an einem oder mehreren Füllstoffen enthalten.

Vorteilhafte Kapselfüllungen zeichnen sich durch Volumenverhältnisse von
- flüssigem Öl bzw. flüssiger Lipidmischung zu
- einer oder mehreren abrasiv wirkenden Festkörpersubstanzen
aus, die aus dem Bereich von 70 : 30 bis 40 : 60 gewählt werden.

Zur Herstellung der erfindungsgemäßen kapselförmigen Zubereitungen werden die Hülle und die Füllung zunächst getrennt voneinander hergestellt. Das Überziehen der Füllung mit dem Hüllmaterial kann, unabhängig von der Zusammensetzung des Füllmaterials oder der Hülle, auf verschiedene Arten erfolgen.

Beispielsweise kann das Füllmaterial eingefroren und dann in aufgeschmolzenes Hüllmaterial, getaucht werden, wodurch sich auf dem Füllmaterial eine feste, geschlossene Hülle bildet.

Es können aber auch aus geschmolzenem Hüllmaterial Hohlkugeln gegossen werden, die durch ein Loch in der Kugelwand ggf. mit Füllmaterial befüllt werden. Danach wird das Loch durch einen Stopfen aus Hüllmaterial verschlossen.

Eine weitere Möglichkeit die Herstellung über das sogenannte One-Shot-Verfahren. Dabei werden Hüll- und Füllmaterial gleichzeitig gegossen. Die Gießmaschine dosiert beide Massen aus dem geteilten Voratsbehälter durch eine kozentrische Ringdüse in eine Blisterform. Zuerst startet die Hüllmasse über den Ringspalt und dann folgt mit kurzer Verzögerung die Füllung über die innere Düse. So kann in einem Schritt die fertige Kapsel gegossen werden. Dieses Verfahren ist das erfindungsgemäß bevorzugte Herstellungsverfahren.

Es ist auch möglich, zunächst halbe Hohlkugeln zu gießen, diese ggf. zu befüllen, zur Deckung zu bringen und abschließend durch thermische Behandlung miteinander zu verschmelzen. Darüber hinaus können zwei solcher Halbkugeln hergestellt werden, wobei eine oder beide ein Loch zu späteren Befüllung aufweisen, dann zu einer Hohlkugel verschweißt und abschließend durch das Füllloch befüllt werden, dass dann wie oben beschrieben verschlossen wird.

Es ist erfindungsgemäß vorteilhaft, wenngleich nicht zwingend, die Peelingkapseln nach bekannten Verfahren zu dragieren. Erfindungsgemäß vorteilhaft wird das Dragiermaterial aus der Gruppe der üblicherweise dafür verwendeten Zucker gewählt, aber auch übliche Dragierlacke, z.B. auf der Basis von Polymethacrylaten od. Methylcellulose oder eine Salzkruste können eingesetzt werden.

Zur Verbesserung der Lagerstabilität und Bruchfestigkeit können Polymere auf die Hülle aufgetragen werden. Geeignete Polymere sind Celluloseether, Polyvinylpyrrolidon, Polyacrylate oder Polymethacrylate, sowie Eudragit.

Ferner kann es vorteilhaft sein die Kapseln mit Polier- oder Hartwachsen zu überziehen. Dazu zählen vor allem Schellack-, Camauba- oder Bienenwachs sowie weitere Lackwachse.

Unter Dragieren versteht man das Umhüllen eines Kerns mit Zuckerschichten. Heute werden immer häufiger andere Überzugsmaterialien verwendet. Die aufgetragene Schicht entweder aus Zucker (=klassisches Zuckerdragee) oder aus einem anderen Filmbildner (= Filmtabletten). Die Schicht ist meist gefärbt und kann gegebenenfalls noch andere Substanzen enthalten, um die Eigenschaften der fertigen Arzneiform in der gewünschten Weise zu verändern (Geruch, Geschmack, ...)

Zuckerdragierung ist das klassische Dragierverfahren, d.h. das Überziehen der Kerne mit Zuckerlösungen. Hohen Produktionskosten, die Schwierigkeiten den Prozess zu automatisieren) und die lange Herstellungsdauer von bis zu einer Woche pro Charge lassen dieses Verfahren zugunsten der Filmtablette immer weiter zurücktreten.

Beim Kaltdragieren wird die Zuckerlösung bei normaler Zimmertemperatur aufgetragen, beim Warmdragieren (Heißdragieren) wird der erwärmte Zuckersirup verwendet (ca. 50 - 60° C).

Der Vorgang findet in Dragierkesseln statt, in der die Kerne durch Rotation der Trommel ins Rollen gebracht werden. Die Dragierflüssigkeit wird zugesetzt und überzieht nach und nach die Kerne. Gleichzeitig wird vorsichtig getrocknet (Warmluft oder UV-Strahler). Bei wärmeempfindlichen Substanzen kann auch über die Zufuhr von Kaltluft getrocknet werden.

Der Vorgang wird so lange wiederholt bis sich eine ausreichend dicke und stabile Schicht um den Kern gebildet hat. Dies kann bis zu 50 Dargiervorgänge erfordern. Die Kerne erfahren dadurch eine Größen- und Volumenzunahme.

Dragierprozess im Einzelnen:
- Imprägnieren
   zum Schutz vor dem Eindringen der Dragierflüssigkeit in den Kern; z.B. mit Schellacklösungen oder Polymerisaten.
- Andecken
   zum mechanischen Schutz und zum Vorbereiten des Auftragens. Andecksirup enthält neben dem Zucker noch Bindemittel (PVP, Cellulose, usw.)
- Auftragen (bis zu 50 mal)
   Das Auftragen ist der eigentliche Dragiervorgang. Wird bis zum Erreichen der gewünschten Dicke wiederholt.
- Färben
   Zum Färben wird der letzten Auftragsschicht Farbstoff zugesetzt (1 - 3%).
- Glätten
   Der Glättsirup wird aufgetragen, um Unebenheiten zu beseitigen. Langsames Trocknen ist wichtig, daher keine Wärmezufuhr.
- Polieren
   Zum Verbessern des Aussehens wird mit Öl oder Polierwachs in besonderen mit Filz ausgeschlagenen Trommeln ohne Wärmeanwendung laufen gelassen.

### Schnelldragierung

Die Schnelldragierung entspricht in ihren wesentlichen Arbeitsgängen der oben beschriebenen Dragierung. Die Zeitersparnis wird dadurch realisiert, dass man sich mit einer um 70% - 90% geringeren Schichtdicke zufrieden gibt. Ferner werden zum Schnelldragieren Dragieremulsionen verwendet. In wenigen Stunden lassen sich so Dragees herstellen

Nachfolgende Beispiele erläutern die erfindungsgemäßen kapselförmigen Zubereitungen. Die Prozentangaben beziehen sich soweit nichts anderes angegeben auf die Gesamtmasse der Zubereitungen.

### Rezepturbeispiele:

| **Hüllmaterial:** | |
|---|---|
| I. | |
| C18-36 Fettsäure Triglyceride | 12,2 % |
| Hydrogenierte Cocoglyceride | 60,96 % |
| Capryl-/Caprinsäure Triglyceride | 12,2 % |
| Cyclomethicon | 12,2 % |
| Silika Dimethylsilylat | 2,44 % |

| II. | |
|---|---|
| Isopropylstearat | 47,17 % |
| Glycerylstearat | 1 % |
| Polyethylen | 10 % |
| Silika Dimethylsilylat | 5 % |
| Triceteareth-4 Phosphat | 0,3 % |
| Cetylalkohol | 36,5 % |

| **Füllmaterial:** | |
|---|---|
| III. | |
| Jojobaöl | 50 % |
| Kochsalz | 50 % |

| IV. | |
|---|---|
| Stearylalkohol | 0,25 % |
| Bienenwachs | 0,25 % |
| C18-36 Fettsäure Triglyceride | 2,75 % |
| Paraffinöl | 46,55 % |
| Parfum | 0,2 % |
| Meersalz | 50 % |

| V. | |
|---|---|
| Cetylalkohol | 10 % |
| Capryl-/Caprinsäure Triglyceride | 40 % |
| Natriumchlorid | 50 % |

### Herstellung: Beispiel I mit IV:

C₁₈₋₃₆ Fettsäure Triglyceride, Hydrogenierte Cocoglyceride, Capryl-/Caprinsäure Triglyceride und Cyclomethicon auf 80°C erwärmen. Im Anschluss unter Rühren Silica Dimethylsilylat zur geschmolzenen Lipidmischung geben.

Stearylalkohol, Bienenwachs, C18-36 Fettsäure Triglyceride und Paraffinöl ebenfalls schmelzen und mit Meersalz mischen.

Über das One-Shot-Verfahen werden Hüll- und Füllmaterial in einen Schritt zur Kapsel vergossen.

Abschließend werden die Kapseln in einem Dragierkessel mit einer Zuckerlösung oder Wachsdispersion dragiert.

### Herstellung: Beispiel II mit III:

Für die Hüllmasse Isopropylstearat, Glycerylstearat, Triceteareth-4 Phosphat und Cetylalkohol auf 80°C erwärmen. Im Anschluss unter Rühren Silica Dimethylsilylat sowie Polyethylen unter die geschmolzenen Lipidmischung mischen.

Für die Füllung Jojobaöl und Kochsalz im Verhältnis 1:1 mischen.

Über das One-Shot-Verfahen werden Hüll- und Füllmaterial in einen Schritt zur Kapsel vergossen.

Abschließend werden die Kapseln in einem Dragierkessel mit einer Zuckerlösung oder Wachsdispersion dragiert.

### Herstellung: Beispiel I mit V:

C₁₈₋₃₆ Fettsäure Triglyceride, Hydrogenierte Cocoglyceride, Capryl-/Caprinsäure Triglyceride und Cyclomethicon auf 80°C erwärmen. Im Anschluss unter Rühren Silica Dimethylsilylat zur geschmolzenen Lipidmischung geben.

Cetylalkohol und Capryl-/Caprinsäure Triglyceride ebenfalls schmelzen und mit Natriumchlorid mischen.

Über das One-Shot-Verfahen werden Hüll- und Füllmaterial in einen Schritt zur Kapsel vergossen.

Abschließend werden die Kapseln in einem Dragierkessel mit einer Zuckerlösung oder Wachsdispersion dragiert.

## Patentansprüche

1. Kosmetische Peelingzubereitungen in Kapselform aus
- einer Kapselhülle aus, der Gruppe der bei 25°C festen kosmetisch üblichen Wachse, oder der bei 25 °C festen Mischungen aus Wachsen und einer oder mehreren Substanzen gewählt aus der Gruppe der kosmetisch üblichen Lipide, Wachse , Emulgatoren und natürlichen oder synthetischen Polymere,
wobei die Wachse gewählt werden aus aus der Gruppe Cetylpalmitat, Cetylrizinoleat, Bienenwachs, hydrierte Kokosglyceride, Methylpalmitat, Candelillawachs, Camaubawachs, Paraffinwachs, Ceresin, Ozokerit, Myristylmyristat, Tripalmitin, Tribehenin, Glycerylpalmitostearat, hydriertes Rapsöl und/oder C15-C40 Alkylstearylstearat, bevorzugt Ceresin und/oder Ozokerit,
- und einer Kapselfüllung aus einem Öl oder Lipidmischung weiches oder welche bei 25 °C eine Viskosität zwischen 3 und 50 000 mPas, bevorzugt zwischen 1000 und 8000 und ganz besonders bevorzugt zwischen 2000 und 4000 mPas aufweist, und einer oder mehreren abrasiv wirkenden Festkörpersubstanzen, gewählt aus der Gruppe Kochsalz, Steinsalz, Siedesalz, Meersalz oder Himalayasalz.

2. Peelingzubereitungen nach Anspruch 1, **dadurch gekennzeichnet, dass** das Kapselmaterial bis zu einer Temperatur von mindestens 35 °C fest bzw. formstabil ist.

3. Peelingzubereitungen nach einem der vorstehenden Ansprüche, **dadurch gekennzeichnet, dass** sie im wesentlichen kugelförmig sind und einen durchschnittlichen Kapseldurchmesser von 5 bis 50 mm, bevorzugt 8 bis 15 mm aufweisen.

4. Peelingzubereitungen nach einem der vorstehenden Ansprüche, **dadurch gekennzeichnet, dass** das durchschnittliche Kapselvolumen etwa 0,25 bis 25 ml beträgt.

5. Peelingzubereitungen nach einem der vorstehenden Ansprüche, **dadurch gekennzeichnet, dass** die durchschnittliche Dicke der Kapselhülle 0,001 bis 3 mm beträgt.

6. Peelingzubereitungen nach einem der vorstehenden Ansprüche, **dadurch gekennzeichnet, dass** die Kapselfüllungen sich durch Volumenverhältnisse von
- flüssigen Öl bzw. flüssiger Lipidmischung zu
- einer oder mehreren abrasiv wirkenden Festkörpersubstanzen auszeichnen, die aus dem Bereich von 70 : 30 bis 40 : 60 gewählt werden.

7. Peelingzubereitungen nach einem der vorstehenden Ansprüche, **dadurch gekennzeichnet, dass** die Korngröße der eingesetzten abrasiv wirkenden Substanz bzw. Substanzen zwischen 40 und 2000 µm, besonders bevorzugt kleiner 800 µm liegt.

## Claims

1. Cosmetic peeling preparations in capsule form comprising
- a capsule shell from the group of the cosmetically customary waxes that are solid at 25°C, or the mixtures of waxes and one or more substances selected from the group of the cosmetically customary lipids, waxes, emulsifiers and natural or synthetic polymers that are solid at 25°C, where the waxes are selected from the group cetyl palmitate, cetyl ricinoleate, beeswax, hydrogenated coco glycerides, methyl palmitate, candelilla wax, carnauba wax, paraffin wax, ceresin, ozokerite, myristyl myristate, tripalmitin, tribehenin, glycerol palmitostearate, hydrogenated rapeseed oil and/or C15-C40 alkylstearyl stearate, preferably ceresin and/or ozokerite,
- and a capsule filling of an oil or lipid mixture which has a viscosity at 25°C between 3 and 50 000 mPas, preferably between 1000 and 8000 and very particularly preferably between 2000 and 4000 mPas, and one or more abrasive solid-body substances selected from the group crooking salt, rock salt, evaporated salt, sea salt or Himalaya salt.

2. Peeling preparations according to Claim 1, **characterized in that** the capsule material is solid and/or dimensionally stable up to a temperature of at least 35°C.

3. Peeling preparations according to one of the preceding claims, **characterized in that** they are essentially spherical and have an average capsule diameter of from 5 to 50 mm, preferably 8 to 15 mm.

4. Peeling preparations according to one of the preceding claims, **characterized in that** the average capsule volume is about 0.25 to 25 ml.

5. Peeling preparations according to one of the preceding claims, **characterized in that** the average thickness of the capsule shell is 0.001 to 3 mm.

6. Peeling preparations according to one of the preceding claims, **characterized in that** the capsule fillings are **characterized by** volume ratios of
- liquid oil or liquid lipid mixture to
- one or more abrasive solid-body substances which are selected from the range from 70:30 to 40:60.

7. Peeling preparations according to one of the preceding claims, **characterized in that** the particle size of the abrasive substance or substances used is between 40 and 2000 µm, particularly preferably less than 800 µm.

## Revendications

1. Compostions cosmétiques de gommage sous forme de capsules, constituées par
- une enveloppe de capsule du groupe des cires cosmétiquement usuelles, solides à 25°C ou des mélanges solides à 25°C de cires et d'une ou de plusieurs substances choisies dans le groupe des lipides, des cires, des émulsifiants et des polymères naturels ou synthétiques, cosmétiquement usuels, les cires étant choisies dans le groupe formé par le palmitate de cétyle, le ricinoléate de cétyle, la cire d'abeille, les glycérides de coco hydrogénés, le palmitate de méthyle, la cire de candelilla, la cire de caroube, la cire de paraffine, la cérésine, l'ozokérite, le myristate de myristyle, la tripalmitine, la tribéhénine, le palmitostéarate de glycéryle, l'huile de colza hydrogénée et/ou le stéarate de C15-C40-alkylstéaryle, de préférence la cérésine et/ou l'ozokérite,
- et un remplissage de capsule constitué par une huile ou un mélange de lipides qui présente à 25°C une viscosité entre 3 et 50 000 mPa.s, de préférence entre 1000 et 8000 et de manière tout particulièrement préférée entre 2000 et 4000 mPa.s, et par une ou plusieurs substances solides à effet abrasif, choisie(s) dans le groupe formé par le sel de table, le sel gemme, le sel de saline, le sel de mer ou le sel de l'Himalaya.

2. Compositions de gommage selon la revendication 1, **caractérisées en ce que** le matériau de la capsule est solide ou présente une forme stable jusqu'à une température d'au moins 35°C.

3. Compositions de gommage selon l'une quelconque des revendications précédentes, **caractérisées en ce qu'**elles sont sensiblement sphériques et présentent un diamètre moyen de capsule de 5 à 50 mm, de préférence de 8 à 15 mm.

4. Compositions de gommage selon l'une quelconque des revendications précédentes, **caractérisées en ce que** le volume moyen des capsules est d'environ 0,25 à 25 ml.

5. Compositions de gommage selon l'une quelconque des revendications précédentes, **caractérisées en ce que** l'épaisseur moyenne de l'enveloppe de la capsule est de 0,001 à 3 mm.

6. Compositions de gommage selon l'une quelconque des revendications précédentes, **caractérisées en ce que** les remplissages de capsule se caractérisent par des rapports volumiques
- d'huile liquide ou de mélange liquide de lipides à
- une ou plusieurs substance(s) solide(s) à effet abrasif
qui sont choisis dans la plage de 70:30 à 40:60.

7. Compositions de gommage selon l'une quelconque des revendications précédentes, **caractérisées en ce que** la granulométrie de la ou des substance(s) à effet abrasif utilisée(s) se situe entre 40 et 2000 µm, en étant de manière particulièrement préférée inférieure à 800 µm.
